# EUROPEAN PATENT APPLICATION

(11) **EP 3 932 435 A1**
(43) Date of publication of application: **05.01.2022**
(21) Application number: 21183277.9
(22) Date of filing: 01.07.2021
(51) Int. Cl.: A61L 2/10, A61L 2/24

(54) **SANITATIONS SYSTEMS FOR AIRCRAFT**

(30) Priority: 01.07.2020 US 202063046991 P; 30.06.2021 US 202117364503
(71) Applicant: B/E Aerospace, Inc., Winston-Salem, NC 27105 (US)
(72) Inventor: BURD, Peter, Burry Port, SA 160 RB (GB)
(74) Representative: Dehns

(57) **Abstract**

A sanitization system (100) can include a housing (101) defining an interior volume (103), and one or more FAR-UVC light sources (105) disposed within the housing and configured to irradiate at least a portion of the interior volume. The interior volume can be configured to receive one or more user hands or portions thereof to sanitize the one or more user hands and/or portions thereof.

## Description

### CROSS-REFERENCE TO RELATED APPLICATIONS

This application claims priority to and the benefit of U.S. Provisional Application No. 63/046,991, filed July 1, 2020.

### FIELD

This disclosure relates to sanitation systems, e.g., for aircraft.

### BACKGROUND

The risk of cross contamination of viral pathogens such as COVID-19 is increased in situations where there are a number of high incidence touch points, e.g., in an aircraft galley and around the passenger cabin. Protective gloves have to be frequently disposed of, and to be truly effective, hand sanitizing gel should be used after every touching incident. As a result a large quantity of personal protective equipment (PPE) needs to be transported to avoid shortages.

Conventional methods and systems have generally been considered satisfactory for their intended purpose. However, there is still a need in the art for improved sanitation systems. The present disclosure provides a solution for this need.

### SUMMARY

A sanitization system can include a housing defining an interior volume, and one or more FAR-UVC light sources disposed within the housing and configured to irradiate at least a portion of the interior volume. The interior volume can be configured to receive one or more user hands or portions thereof to sanitize the one or more user hands and/or portions thereof.

The one or more FAR-UVC light sources can include at least one FAR-UVC light source disposed on each internal surface of each wall of the housing. The housing can define an enclosed chamber having an opening. For example, the housing can include five walls.

The system can include a proximity sensor disposed on or within the housing and configured to detect when a user has inserted a hand and/or portion thereof into the enclosed chamber. The system can include a control module operatively connected to the proximity sensor and to each FAR-UVC light sources to turn on each FAR-UVC light source when a user's hand and/or portion thereof is detected by the proximity sensor.

The system can include one or more visible lights disposed within the internal volume to indicate that the device is active when in use, wherein the control module is configured to turn on the one or more visible lights when the one or more FAR-UVC light sources are turned on. Any suitable visible wavelength is contemplated herein (e.g., about 405 nm UV).

The one or more FAR-UVC light sources can be a FAR-UVC LED that emits light between about 207 nm to about 222 nm. Any suitable band configured to inactivate viruses, bacteria, and/or other microbes without posing a risk to humans is contemplated herein.

In accordance with at least one aspect of this disclosure, an aircraft can include a sanitization system as disclosed herein, e.g., as described above. The sanitization system can be in any suitable location (e.g., a lavatory, a galley).

In accordance with at least one aspect of this disclosure, a method can include detecting a presence of an object in a chamber, and activating a FAR-UVC light source in response to irradiate the object in the chamber. The object can be one or more hands and/or a portion thereof. Activating the FAR-UVC light source can include activating for at least a period of time sufficient to cause viral, bacterial, or other microbial inactivation.

These and other features of the embodiments of the subject disclosure will become more readily apparent to those skilled in the art from the following detailed description taken in conjunction with the drawings.

### BRIEF DESCRIPTION OF THE DRAWINGS

So that those skilled in the art to which the subject disclosure appertains will readily understand how to make and use the devices and methods of the subject disclosure without undue experimentation, embodiments thereof will be described in detail herein below, by way of example only and with reference to certain figures, wherein:
Fig. 1 is a schematic diagram of an embodiment of a sanitization system in accordance with this disclosure;
Fig. 2 is a view of an embodiment of a sanitization system in accordance with this disclosure, shown in an on state; and
Fig. 3 illustrates an embodiment of a sanitization system in use.

### DETAILED DESCRIPTION

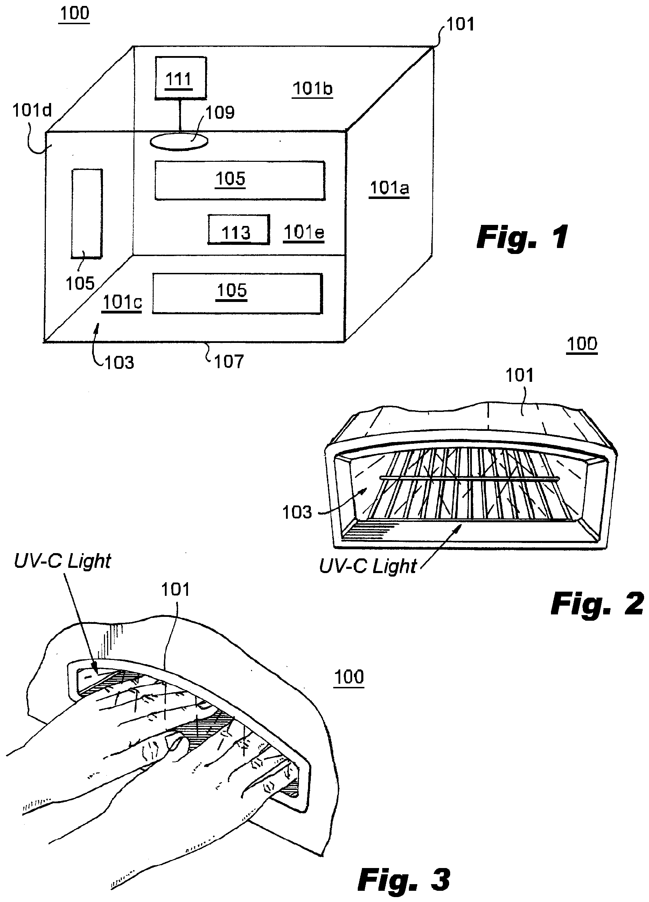

Reference will now be made to the drawings wherein like reference numerals identify similar structural features or aspects of the subject disclosure. For purposes of explanation and illustration, and not limitation, an illustrative view of an embodiment of a sanitization system in accordance with the disclosure is shown in Fig. 1 and is designated generally by reference character 100. Other embodiments and/or aspects of this disclosure are shown in Figs. 2 and 3. Certain embodiments described herein can be used to irradiate a user's hands without hurting the user while sanitizing the user's hands, for example. Any other suitable use is contemplated herein.

Referring to Figs. 1 and 2, a sanitization system 100 can include a housing 101 defining an interior volume 103, and one or more FAR-UVC light sources 105 disposed within the housing 101 and configured to irradiate at least a portion of the interior volume 103 (e.g., the entirety). The interior volume 103 can be configured (e.g., sized and shaped) to receive one or more user hands or portions thereof to sanitize the one or more user hands and/or portions thereof (e.g., as shown in Fig. 3C).

The one or more FAR-UVC light sources 105 can include at least one FAR-UVC light source 105 disposed on each internal surface of each wall 101a, b, c, d, e of the housing 101, for example (e.g., as shown in Fig. 1). As shown , the housing 101 can define an enclosed chamber having an opening 107 (e.g., a single opening). For example, the housing 101 can include five walls 101a, b, c, d, e (and the opening 107).

The system 100 can include a proximity sensor 109 disposed on or within the housing 101 (e.g., at or proximate to the opening 107) and configured to detect when a user has inserted a hand and/or portion thereof into the enclosed chamber (e.g., as shown in Fig. 3C). Any suitable proximity sensor (and/or any components thereof) known to those having ordinary skill in the art is contemplated herein (e.g., similar to a touchless faucet system).

The system 100 can include a control module 111 operatively connected to the proximity sensor 109 and to each FAR-UVC light source 105 to turn on each FAR-UVC light source 105 when a user's hand and/or portion thereof is detected by the proximity sensor 109. The control module 111 can include any suitable hardware and/or software module(s) configured to perform any suitable function (e.g., as disclosed herein or otherwise). Any suitable power supply (e.g., corded, battery, etc.) for powering the device 100 is contemplated herein.

The system 100 can include one or more visible lights 113 disposed within the internal volume 103 to indicate that the device 100 is active when in use. Any other suitable location is contemplated herein (e.g., external of the device 100). The control module 111 can be configured to turn on the one or more visible lights 113 when the one or more FAR-UVC light sources 105 are turned on. Any suitable visible wavelength is contemplated herein (e.g., about 405 nm UV).

The one or more FAR-UVC light sources 105 can be a FAR-UVC LED that emits light between about 207 nm to about 222 nm. Any other suitable source is contemplated herein. Any suitable light band configured to inactivate viruses, bacteria, and/or other microbes without posing a risk to humans is contemplated herein.

In accordance with at least one aspect of this disclosure, an aircraft (not shown) can include a sanitization system (e.g., system 100) as disclosed herein, e.g., as described above. The sanitization system can be in any suitable location of the aircraft (e.g., a lavatory, a galley).

In accordance with at least one aspect of this disclosure, a method can include detecting a presence of an object in a chamber, and activating a FAR-UVC light source in response to irradiate the object in the chamber. The object can be one or more hands and/or a portion thereof. Activating the FAR-UVC light source can include activating for at least a period of time sufficient to cause viral, bacterial, or other microbial inactivation.

Embodiments can include a FAR-UVC hand sanitizer for passenger aircraft, for example. Embodiments can include a box or other suitable chamber open on at least one side to allow the user to insert their bare or gloved hands. The chamber can be internally equipped with an array of FAR-UVC and visible UV light LEDs on the remaining internal sides, FAR-UVC can be a short wave UV in the 207 to 222 nm band that does not pose a human skin or eye exposure risk, but is extremely effective against viruses. The device can also be equipped with visible light UV LEDs in the 405 nm range to provide the user with a reference and reassurance that the device is operational. To sanitize their hands the user can insert them into the open slot rotating them back and forth for about 20 to about 60 seconds. Tuning the device on could be done by a manual switch (e.g., touchless) or a proximity sensor and can include an auto-off function (e.g., turn off after about 60 seconds).

Installing an embodiment of a device, either as an integrated part of a galley, lavatory, or in the passenger cabin as a sanitizing station can significantly reduce the volume of PPE required on an aircraft. Embodiments can also provide a quick, easy to use, and effective method of deactivating a virus and preventing cross commination between the cabin crew, passengers and high touch surfaces. Using the device can be similar to using a washroom hand drier, and integrating both FAR-UVC and visible UV can aid effectiveness.

A problem in aircraft can be viral contamination of flight attendants and passengers hands as a result of touching infected surfaces or objects. Protective gloves can be used once, and then have to be disposed of. Sanitizing gel can exacerbate some skin conditions. Significant stock needs to be carried as well, which is heavy. As a solution to these problems, embodiments can provide a modular sanitation station consisting of a cavity with FAR-UV LED lamps on all internal sides. The operating procedure can be similar to using a washroom drier, and a user deactivates the virus by inserting their bare or gloved hands inside. Embodiments eliminate the need to carry significant quantities of consumable gel on board which saves weight and cost, and also cannot run out of stock in flight. Embodiments can reduce the volume of potentially contaminated disposable PPE trash, can be installed on galleys, lavatories, etc., or as a standalone cleansing station. Embodiments are easy to use, require very low power consumption, can provide auto power off when not in use, can provide a safe timed cleaning cycle, and could be available to both flight crew and passengers.

As will be appreciated by those skilled in the art, aspects of the present disclosure may be embodied as a system, method or computer program product. Accordingly, aspects of this disclosure may take the form of an entirely hardware embodiment, an entirely software embodiment (including firmware, resident software, micro-code, etc.), or an embodiment combining software and hardware aspects, all possibilities of which can be referred to herein as a "circuit," "module," or "system." A "circuit," "module," or "system" can include one or more portions of one or more separate physical hardware and/or software components that can together perform the disclosed function of the "circuit," "module," or "system", or a "circuit," "module," or "system" can be a single self-contained unit (e.g., of hardware and/or software). Furthermore, aspects of this disclosure may take the form of a computer program product embodied in one or more computer readable medium(s) having computer readable program code embodied thereon.

Any combination of one or more computer readable medium(s) may be utilized. The computer readable medium may be a computer readable signal medium or a computer readable storage medium. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium may be any tangible medium that can contain, or store a program for use by or in connection with an instruction execution system, apparatus, or device.

A computer readable signal medium may include a propagated data signal with computer readable program code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

Program code embodied on a computer readable medium may be transmitted using any appropriate medium, including but not limited to wireless, wireline, optical fiber cable, RF, etc., or any suitable combination of the foregoing.

Computer program code for carrying out operations for aspects of this disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of this disclosure may be described above with reference to flowchart illustrations and/or block diagrams of methods, apparatus (systems) and computer program products according to embodiments of this disclosure. It will be understood that each block of any flowchart illustrations and/or block diagrams, and combinations of blocks in any flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in any flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified herein.

Those having ordinary skill in the art understand that any numerical values disclosed herein can be exact values or can be values within a range. Further, any terms of approximation (e.g., "about", "approximately", "around") used in this disclosure can mean the stated value within a range. For example, in certain embodiments, the range can be within (plus or minus) 20%, or within 10%, or within 5%, or within 2%, or within any other suitable percentage or number as appreciated by those having ordinary skill in the art (e.g., for known tolerance limits or error ranges).

The articles "a", "an", and "the" as used herein and in the appended claims are used herein to refer to one or to more than one (i.e., to at least one) of the grammatical object of the article unless the context clearly indicates otherwise. By way of example, "an element" means one element or more than one element.

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

Any suitable combination(s) of any disclosed embodiments and/or any suitable portion(s) thereof are contemplated herein as appreciated by those having ordinary skill in the art in view of this disclosure.

The embodiments of the present disclosure, as described above and shown in the drawings, provide for improvement in the art to which they pertain. While the subject disclosure includes reference to certain embodiments, those skilled in the art will readily appreciate that changes and/or modifications may be made thereto without departing from the spirit and scope of the subject disclosure.

## Claims

1. A sanitization system (100) comprising:
a housing (101) defining an interior volume (103); and
one or more FAR-UVC light sources (105) disposed within the housing and configured to irradiate at least a portion of the interior volume (103), wherein the interior volume (103) is configured to receive one or more user hands or portions thereof to sanitize the one or more user hands and/or portions thereof.

2. The system of claim 1, wherein the one or more FAR-UVC light sources include at least one FAR-UVC light source (105) disposed on each internal surface of each wall (101a-e) of the housing.

3. The system of claim 1 or 2, wherein the housing defines an enclosed chamber having an opening (107).

4. The system of any preceding claim, wherein the housing includes five walls.

5. The system of claim 3, further comprising a proximity sensor (109) disposed on or within the housing (101) and configured to detect when a user has inserted a hand and/or portion thereof into the enclosed chamber.

6. The system of claim 5, further comprising a control module (111) operatively connected to the proximity sensor (109) and to each FAR-UVC light sources (105) to turn on each FAR-UVC light source when a user's hand and/or portion thereof is detected by the proximity sensor.

7. The system of any preceding claim, further comprising one or more visible lights (113) disposed within the internal volume to indicate that the device is active when in use, wherein the control module is configured to turn on the one or more visible lights (113) when the one or more FAR-UVC light sources are turned on.

8. The system of any preceding claim, wherein the one or more FAR-UVC light sources is a FAR-UVC LED that emits light between about 207 nm to about 222 nm.

9. An aircraft, comprising:
the sanitization system (100) according to any preceding claim.

10. A method comprising:
detecting a presence of an object in a chamber; and
activating a FAR-UVC light source (105) in response to irradiate the object in the chamber.

11. The method of claim 10, wherein the object is one or more hands and/or a portion thereof.

12. The method of claim 10 or 11, wherein activating the FAR-UVC light source (105) includes activating for at least a period of time sufficient to cause viral, bacterial, or other microbial inactivation.
